# EUROPÄISCHE PATENTSCHRIFT

(11) **EP 3 479 114 B1**
(45) Veröffentlichungstag und Bekanntmachung des Hinweises auf die Patenterteilung: **19.08.2020**
(21) Anmeldenummer: 16738705.9
(22) Anmeldetag: 04.07.2016
(51) Int. Cl.: G01N 33/00, F04B 43/04, F04B 45/047

(54) **GERÄT MIT MIKROFLUIDAKTOR**
DEVICE WITH MICROFLUIDIC ACTUATOR
APPAREIL À ACTIONNEUR MICROFLUIDIQUE

(43) Veröffentlichungstag der Anmeldung: 08.05.2019
(73) Patentinhaber: Fraunhofer Gesellschaft zur Förderung der angewandten Forschung E.V., 80686 München (DE)
(72) Erfinder: RICHTER, Martin, 81677 München (DE); WALD, Christian, 80689 München (DE); CONGAR, Yuecel, 86807 Buchloe (DE)
(74) Vertreter: Stöckeler, Ferdinand
(86) Internationale Anmeldenummer: PCT/EP2016/065694
(87) Internationale Veröffentlichungsnummer: WO 2018/006932

(56) Entgegenhaltungen:
- EP-A1- 2 733 484
- WO-A2-01/30497
- WO-A2-01/94920
- US-A1- 2005 229 675

## Beschreibung

Die Erfindung betrifft ein Gerät mit den Merkmalen von Anspruch 1.

Heutzutage werden immer mehr Sensoren genutzt. Viele dieser Sensoren messen Umweltparameter, wie z.B. CO₂, Feuchtigkeit, Temperatur oder Rauch. Allerdings messen diese Sensoren naturgemäß nicht die Parameter des Raumes, sondern die Parameter innerhalb des jeweiligen Gerätes in das sie integriert sind. Mit komplizierten Algorithmen wird dann versucht, aus dem Messsignal des Sensors näherungsweise auf die Parameter in der Raumluft zu schließen, was aber nur teilweise gelingt.

Eine Detektion von Gasen wie CO, N₂O oder VOC sowie von Wasserinhalten und der Gaszusammensetzung in unserer Umgebung liegt im Interesse vieler Menschen.

Außerdem möchten die meisten Menschen luftverschmutzende Gase, Feinstaub und allergene Partikel wie Pollen augenblicklich an ihrem Standort erfassen oder Frühwarnungen vor allergenen Substanzen und gesundheitsgefährdenden Gasen erhalten. Darüber hinaus ist auch die Detektion von Gerüchen, wie z.B. Atemalkohol, Mundgeruch und vielen anderen Gerüchen, von Interesse.

Ein weiteres Anwendungsgebiet der Messung von Umgebungsparametern ist die sogenannte Context Awareness von Mobilgeräten. Hierbei reagiert das Gerät eigenständig auf seine direkte Umgebung durch Ein- oder Abschalten oder Anpassen von Funktionen wie beispielsweise akustische Signaltöne oder Vibrationsalarme und Sendefunktionen oder das Abschalten von Stromverbrauchern zur Schonung der Batterielebenszeit. Beispiele hierfür sind die Erkennung von Innen- und Außenräumen oder Flugzeugen, die Anwesenheit anderer Personen oder die Trageart wie beispielsweise körperfern in der Hand- oder Aktentasche oder körpernah in der Hemd- oder Hosentasche.

Sensoren stehen für viele der genannten Anwendungen zur Verfügung. Obwohl viele der verfügbaren Sensoren (z.B. für Luftfeuchtigkeit, flüchtige organische Verbindungen VOC, CO oder NO) oder der Sensoren, die auf metallorganischen, optischen oder Resonanzprinzipien basieren, Formfaktoren und eine geeignete Kosteneffizienz für die Anwendung mit portablen Geräten aufweisen, ist die kommerzielle Nutzung nur sehr eingeschränkt möglich, da das Gas, der Geruch oder der Feinstaub nicht bei allen Sensorprinzipien schnell und zuverlässig zu dem Sensor in einem portablen Gerät gebracht werden kann.

Dies ist jedoch erforderlich, um dem Benutzer ein aussagekräftiges und schnelles Messergebnis zur Verfügung zu stellen. Zuverlässige Daten für Gasmessungen können aussagekräftige Gasübersichten der Umgebung zur Verfügung stellen, z.B. Luftverschmutzung entlang von Straßen bzw. in großen Städten oder Pollenkarten für Allergiker.

Sensoren werden in einigen Fällen in sperrige, eigenständige Geräte verbaut. In einigen Fällen müssen bei diesen Geräten Mundstücke verwendet werden, was viele Menschen nicht mögen. Ein zusätzliches Problem ist, dass eigenständige Geräte mit Sensoren Anzeige- und Rechenfähigkeiten benötigen, was zusätzliche Kosten verursacht.

Ein derartiges spezialisiertes portables Gasmessgerät ist aus der WO 2015/104221 A1 bekannt. Die Druckschrift bezieht sich auf eine Sensoreinheit für ein Gasmessgerät zum Detektieren eines Gases. Das transportable Gasmessgerät dient der Überwachung von Gasen und Dämpfen, insbesondere von toxischen Gasen im industriellen Umfeld. Allgemein geht es um die Änderung einer physikalisch-chemischen Eigenschaft des Rezeptors, welcher auf molekularer Ebene mit Analytmolekülen wechselwirkt. Die WO 2015/104221 A1 beschreibt diesbezüglich eine Zusammenstellung aus einem druckdichten Messkanal, einem Gaseinlass, einem Gasauslass, einer Pumpeinheit zum Evakuieren, einem Gassensor, einer Heizeinheit für den Gassensor und einer Sensoreinheit mit Regenerations- & Messmodus.

Wie eingangs erwähnt, ist das aus der WO 2015/104221 A1 bekannte Gasmessgerät eine spezialisierte Vorrichtung, die ausschließlich zum Messen von Gasen in der Umgebungsluft vorgesehen ist.

Die EP 2 733 484 A1 beschreibt verschiedene Pumpvorrichtungen in einem Gehäuse eines Mobiltelefons. Angesaugte und zu analysierende Luft kann mittels Lüfter, mechanischen Drucktastern, Piezos oder Schallwandlern innerhalb einer Leitung bewegt werden.

Die WO 01 / 30497 A2 beschreibt eine Makro-Mesopumpe, die in einem sogenannten "sniffing-mode" betrieben werden kann. Um genügend Analyt-Gas zum Sensor zu befördern, wird die Pumpe im Sinne eines abwechselnden Ein- und Ausatmens mehrfach hintereinander betätigt. Auch die WO 01 / 94920 A2 beschreibt eine derartige Makro-Mesopumpe.

Derartige bekannte Vorrichtungen liefern zwar gute und zuverlässige Messergebnisse. Jedoch benötigen derartige Vorrichtungen eine gewisse Zeit, während der sich die Gassensorik kalibriert, um ein ausreichend zuverlässiges Ergebnis zu liefern. Derartige bekannte Geräte sind daher insbesondere in Fällen, in denen sich die Umgebungsbedingungen rasch ändern, nicht sofort einsatzbereit. Dies ist beispielsweise dann der Fall, wenn das Gerät von einem Außenbereich in einen Innenbereich, z.B. in einen Wohnraum, gebracht wird. Hier benötigen bekannte Vorrichtungen sozusagen eine gewisse Zeit zum "Akklimatisieren" wobei diese Zeit teilweise mehrere Minuten betragen kann.

Nun sind für viele Applikationen von portablen elektronischen Geräten aber schnelle Ansprechzeiten der Gassensoren wünschenswert, etwa bei einer schnellen Personenerkennung durch Gassensoren, bei Brandmeldesensoren oder um zu erkennen wenn eine Person ein Gebäude betritt. Der Hersteller von Gasmessgeräten müsste nun, wenn er diese Erkenntnisse berücksichtigt, seine Sensoren unmittelbar an der Gehäuseoberfläche montieren, um ein schnelles Signal zu erhalten. Dies ist aber aus mehreren Gründen unwirtschaftlich oder unvorteilhaft.

Zum einen erhöht das Anordnen des Sensors auf einer der Platinen im Gehäuse die Montageanforderungen und damit zwangsläufig die damit verbundenen Montagekosten. Außerdem besteht bei einer Anbringung unmittelbar an der Gehäuseoberfläche die Gefahr der Beschädigung des Sensors durch Einwirkung von außen. Wenn der Sensor hingegen außen an dem Gasmessgerät verbaut wird, dann ist das Gehäuse unter Umständen nicht mehr glatt. Darüber hinaus steht, wenn viele Gassensoren und andere Bauelemente (z.B. Platinen, Displays, etc.) nahe am Lufteinlass des Gasmessgeräts montiert werden sollen, unter Umständen nicht genügend Platz zur Verfügung.

In Smartphones war bereits ein Feuchtesensor in Serie. Jedoch hatte dieser Sensor lange Ansprechzeiten im Bereich von mehreren Minuten. Der Sensor befand sich in dem Gehäuse des Smartphones. Das Smartphone-Gehäuse wies eine Gehäuseöffnung auf, durch die Luft zu dem Sensor diffundiert ist. Die Diffusion als Transportmechanismus zwischen der Gehäuseöffnung und dem Sensor war jedoch sehr langsam.

Derzeit arbeiten Sensorentwickler daran, diese Ansprechzeiten zu verkürzen. Eine Lösung sieht vor, den Sensor möglichst nahe an der Öffnung zu positionieren, um den Diffusionsweg und somit die für die Diffusion benötigte Zeit zu verkürzen.

Ein weiterer Lösungsvorschlag sieht vor, mittels Software aus der Anfangssteigung des Sensorsignals den Endwert zu prognostizieren. Somit ist ein anfänglicher Sensorwert ausreichend, um den weiteren Verlauf zu extrapolieren und somit Zeit zu sparen.

Beide Lösungsvorschläge haben jedoch ihre technischen Grenzen. Somit ist die Realisierung einer Sensor-Ansprechzeit von weniger als 20-30 Sekunden nur schwer möglich.

Derzeit besteht ein Trend, dass neben Feuchtesensoren auch weitere Sensoren, die Luftparameter messen, in ein Mobilfunkgerät und andere tragbare Geräte (Wearables, Uhren, etc...) integriert werden.

Bei allen diesen Sensoren ist die Ansprechzeit ein kritischer Parameter, der heute noch nicht zufriedenstellend gelöst ist. Mobilfunkhersteller beispielsweise fordern die Ausgabe eines Sensorwerts bereits innerhalb von ein bis zwei Sekunden nachdem der Benutzer den Wert anfordert. Ohne einen Mikroaktuator bzw. Mikrofluidaktuator, der die Luftprobe aktiv (d.h. mittels Konvektion) zum Sensor pumpt, kann diese Anforderung jedoch nicht zufriedenstellend gelöst werden.

Es wurden bereits Mobilgeräte mit einer Mikropumpe vorgestellt, bei der eine Silizium-Mikromembranpumpe die Luftzufuhr zu dem Sensor übernimmt. Damit können schnelle Ansprechzeiten realisiert werden. Diese Technik hat aber einige Schwierigkeiten.

Beispielsweise sind die hierbei zum Einsatz kommenden Mikropumpen unidirektional. Das heißt, das zu pumpende Medium muss, nachdem es den Sensor erreicht hat, wieder auf einem anderen Weg das Mobilgerät verlassen. Entweder die Luft wird in das Mobilgerät hinein gepumpt (was andere Nachteile hat), oder es wird eine zweite Gehäuseöffnung benötigt, um die Luft wieder aus dem Gerät heraus zu befördern.

Auch hierfür sind bereits Lösungsansätze, und zwar in Form von bidirektionalen Mikropumpen, bekannt. Bidirektionale Mikropumpen (z.B. eine von der Fraunhofer EMFT entwickelte Mikroperistaltikpumpe mit drei Piezoaktoren) saugen Luft in einem Ansaugtrakt an und stoßen diese Luft in einem Auslasstrakt wieder aus. Bidirektionale Mikropumpen sind aber wesentlich größer als unidirektionale Mikropumpen, und somit auch in der Herstellung entsprechend teurer.

Hinzu kommt, dass alle mechanischen Mikropumpen und Ventile bewegliche Teile aufweisen. Falls Partikel angesaugt werden, können sich diese in den Mikroventilen verklemmen, was zu einem Leistungsabfall der Mikropumpe, oder sogar zum Ausfall der Mikropumpe führen kann.

Es ist eine Aufgabe der vorliegenden Erfindung, ein Gerät, insbesondere Mobilgerät, mit einem Sensor bereitzustellen, das die oben genannten Probleme adressiert und löst.

Diese Aufgabe wird erfindungsgemäß mit einem Gerät mit den Merkmalen von Anspruch 1 gelöst.

Das erfindungsgemäße Gerät weist unter anderem eine Öffnung auf, die eine Fluidverbindung zwischen einem Fluidkanal in dem Gerät und Umgebungsluft definiert. Die Öffnung ist eine Gehäuseöffnung, d.h. eine Öffnung in dem Gehäuse des Geräts. Die Öffnung verbindet das Innere des Geräts mit der Umgebung, d.h. es kann Fluid, und insbesondere Luft, durch diese Öffnung von außen in das Gerät einströmen. Genauer gesagt verbindet die Öffnung die Umgebung mit dem im Inneren des Geräts angeordneten Fluidkanal. Der Fluidkanal kann beispielsweise ein dünnes Röhrchen, ein Schlauch oder dergleichen sein. Der Fluidkanal kann starr, vorzugsweise aber flexibel sein. Das erfindungsgemäße Gerät weist außerdem einen mit dem Fluidkanal gekoppelten Sensor auf, der ausgebildet ist, um zumindest einen Bestandteil der Umgebungsluft zu erfassen. Der Sensor ist fluidisch mit dem Fluidkanal gekoppelt, d.h. der Sensor kann in oder an dem Fluidkanal angeordnet sein, sofern das durch den Fluidkanal strömende Fluid zumindest teilweise auch zu dem Sensor bzw. durch den Sensor strömt. Ferner weist das erfindungsgemäße Gerät einen dem Sensor nachgeschalteten Mikrofluidaktuator auf. Dieser Mikrofluidaktuator ist ausgebildet, um im Saughub Fluid durch den Fluidkanal anzusaugen und in Richtung des Sensors zu befördern und im Druckhub das angesaugte Fluid durch ebendiesen Fluidkanal wieder zurück in Richtung der Öffnung zu befördern. Der Mikrofluidaktuator ist ein Mittel zum Ansaugen (Saughub) und Ausstoßen (Druckhub) von Fluid. Dabei saugt der Mikrofluidaktuator Fluid (z.B. Umgebungsluft) von außen durch die Öffnung in den Fluidkanal. Das einströmende Fluid strömt den mit dem Fluidkanal gekoppelten Sensor an. Nach dem Ansaugen stößt der Mikrofluidaktuator das angesaugte Fluid wieder aus, und zwar wieder durch den Fluidkanal, nur eben in die entgegengesetzte Richtung, sodass das angesaugte Fluid durch die Öffnung wieder zurück in die Umgebung ausgestoßen wird. Der Mikrofluidaktuator schiebt also sozusagen ein Fluidvolumen, das sich in dem Fluidkanal befindet, hin und her. Erfindungsgemäß ist das Volumen des Fluidkanals (bzw. das Volumen des in dem Fluidkanal befindlichen Fluids) zwischen dem Sensor und der Öffnung genauso groß oder kleiner als das Hubvolumen, das der Mikrofluidaktuator mit einem einzelnen Saughub fördern kann. Das heißt, der Mikrofluidaktuator kann mit einem einzelnen Saughub soviel Fluidvolumen in dem Fluidkanal bewegen, dass das von außen durch die Öffnung anzusaugende Fluid die gesamte Länge von der Öffnung bis zu dem Sensor zurücklegt. Erfindungsgemäß ist das Hubvolumen des Mikrofluidaktuators um mindestens das 2,5-fache größer als das Volumen eines Fluidkanalabschnitts zwischen dem Sensor und der Gehäuseöffnung. Außerdem handelt es sich bei dem Mikrofluidaktuator um einen Membranaktuator mit einem Träger und einer daran angeordneten auslenkbaren Membran, wobei die Membran mechanisch vorgespannt ist, sodass die Membran in einer unbetätigten Ruheposition von dem Träger beabstandet ist und sich bei Betätigung zu dem Träger hin bewegt.

Es ist denkbar, dass sich der Fluidkanal zwischen der Öffnung und dem Mikrofluidaktuator erstreckt, und der Fluidkanal, der mit dem Fluidkanal gekoppelte Sensor und der Mikrofluidaktuator zusammen eine fluiddichte Anordnung bilden, die gegenüber dem Innenraum des Mobilgeräts abgedichtet ist. Dies begünstigt die Genauigkeit bei der Verschiebung des in dem Fluidkanal befindlichen geringen Fluidvolumens (im Bereich von Mikrolitern). Die fluiddichte Anordnung sollte im Wesentlichen dicht sein gegenüber dem restlichen Mobilgerät, so dass sich beispielsweise durch die Öffnung des Mobilgeräts angesaugte Umgebungsluft nicht mit der im Innenraum des Mobilgeräts befindlichen Geräteluft vermischt. Gewisse kleine Gas-Leckraten wären akzeptabel, sofern diese nicht das Ergebnis der Sensorik verfälschen.

Es ist vorstellbar, dass das Hubvolumen des Mikrofluidaktuators um mindestens das 10-fache, größer ist als das Volumen des Fluidkanals zwischen dem Sensor und der Öffnung. So kann sichergestellt werden, dass beim Ansaugen der Umgebungsluft mittels des Mikrofluidaktuators ausreichend Fluid durch den Fluidkanal strömt und den in dem Fluidkanal angeordneten Sensor erreicht.

Gemäß einer Ausführungsform ist das Mobilgerät ein Mobiltelefon und die in dem Mobiltelefon vorgesehene Öffnung ist eine Mikrofonöffnung. Somit kann auf das Vorsehen einer zusätzlichen Gehäuseöffnung verzichtet werden, da die Mikrofonöffnung in einem Mobiltelefon bereits vorhanden ist.

Gemäß einer weiteren Ausführungsform kann der Sensor ausgebildet sein, um mindestens einen Umgebungsluftbestandteil aus der Gruppe von Kohlenstoffmonoxid (CO), Kohlendioxid (CO₂), Stickstoff (N₂) Distickstoffmonoxid (N₂O), flüchtige organische Verbindungen (VOC), Luftfeuchtigkeit und Feinstaub zu erfassen. Das heißt, der Sensor kann beispielsweise erfassen, ob ein solcher Umgebungsluftbestandteil vorhanden ist, und der Sensor kann außerdem die Konzentration dieses Umgebungsluftbestandteils ermitteln.

Gemäß einer denkbaren Ausführungsform kann die Membran piezoelektrisch oder elektromagnetisch oder elektrostatisch oder mittels elektroaktiven Polymeraktoren oder mittels einem Element aufweisend eine Formgedächtnislegierung betätigbar sein. Mittels Hubbewegungen der Membran wird Fluid gefördert. Die Membran kann aus ihrer Ruheposition mittels der vorgenannten Betätigungsmittel ausgelenkt werden, um eine Hubbewegung (Saughub und/oder Druckhub) auszuführen und Fluid durch den Fluidkanal zu fördern. Ein derartig einfach aufgebauter Membranaktuator ist vorteilhaft gegenüber z.B. (Mikro-)Membranpumpen, da Pumpen einen Einlass und einen Auslass aufweisen, die wiederum mit Ein- und Auslassventilen im richtigen Moment geöffnet bzw. verschlossen werden müssen. Das heißt, Pumpen weisen in der Regel einen einlassseitigen sowie einen auslassseitigen Fluidkanal auf. Der erfindungsgemäße Membranaktuator hingegen weist lediglich einen einzigen Fluidkanal auf, durch den im Saughub das Fluid einströmt und im Druckhub das Fluid wieder ausströmt.

Es ist denkbar, dass das Gerät ein Mobiltelefon mit einem Vibrationsalarmmotor ist und der Mikrofluidaktuator ein Membranaktuator mit einer auslenkbaren Membran ist, wobei die Membran mittels des Vibrationsalarmmotors betätigbar ist. Somit kann der bereits vorhandene Vibrationsalarmmotor zur Betätigung der Membran verwendet werden, d.h. es kann auf separate Betätigungsmittel verzichtet werden. Dies spart wiederum Bauraum, was bei Geräten, insbesondere Mobilgeräten, heutzutage ein wichtiges Kriterium ist.

Gemäß einer Ausführungsform kann der Mikrofluidaktuator ein Membranaktuator mit einer auslenkbaren Doppelmembran sein. So kann die mechanische Spannung, die beim Betätigen der Membran auf selbige wirkt, geringer gehalten werden im Vergleich zu einer Einzelmembran.

Erfindungsgemäß ist die Membran mechanisch vorgespannt, sodass die Membran in einer unbetätigten Ruheposition von dem Träger beabstandet ist und sich bei Betätigung zu dem Träger hin bewegt. Die Membran kann beispielsweise eine vorgespannte Metallmembran, wie z.B. eine Edelstahlmembran, sein. Aufgrund der Vorspannung ist die Membran gewölbt, sodass sie in ihrer Ruheposition von dem Träger beabstandet ist. Die sich zwischen dem Träger und der vorgespannten Membran ausbildende Kavität kann beispielsweise das Hubvolumen des Mikrofluidaktuators bestimmen. Im Falle einer beispielhaften elektrischen Betätigung der Membran kann durch Anlegen einer (z.B. positiven) elektrischen Spannung die Membran betätigt werden, sodass sich die Membran in Folge dessen in Richtung des Trägers bewegt. Die Membran führt in diesem Fall einen Druckhub aus und der Mikrofluidaktuator fördert Fluid, das sich in diesem Fall von dem Mikrofluidaktuator weg bewegt. Wenn die elektrische Spannung nicht weiter angelegt wird (oder z.B. eine negative elektrische Spannung angelegt wird), dann kehrt die Membran wieder in ihre vorgespannte Ruheposition zurück, d.h. sie bewegt sich von dem Träger weg. Dementsprechend bildet sich in dem Fluidkanal ein Unterdruck aus, der das Fluid ansaugt. Das heißt der Mikrofluidaktuator führt einen Saughub aus und fördert Fluid, das sich in diesem Fall zu dem Mikrofluidaktuator hin bewegt.

Es ist denkbar, dass die Membran einschließlich des Trägers eine Höhe von 0,50 mm oder weniger aufweist. Eine derartige Höhe ist vorteilhaft, um den Mikrofluidaktuator in einem Gehäuse insbesondere eines Mobilgeräts zu integrieren.

Gemäß einer Ausführungsform kann das Gerät zwei oder mehr Sensoren aufweisen, die in Strömungsrichtung nacheinander in Reihe angeordnet sind. Eine Reihenanordnung bedeutet, dass die einzelnen Sensoren in Strömungsrichtung räumlich nacheinander in dem Fluidkanal angeordnet sind, sodass ein durch den Fluidkanal strömendes Fluid zeitlich nacheinander durch den jeweiligen Sensor fließt. Die einzelnen Sensoren können jeweils andere Parameter bzw. Bestandteile des angesaugten Fluids messen.

Gemäß einer weiteren denkbaren Ausführungsform kann das Gerät zwei oder mehr Sensoren aufweisen, die in Strömungsrichtung parallel zueinander angeordnet sind. Eine Parallelschaltung einzelner Sensoren bedeutet, dass der Fluidkanal aufzweigt und in jedem Zweig mindestens ein Sensor angeordnet ist. Ein durch den Fluidkanal strömendes Fluid fließt hierbei etwa zeitgleich durch den jeweiligen Sensor. Bei der Parallelschaltung ist es sinnvoll, wenn die Strömungswiderstände aller Sensoren in etwa gleich sind. Eine Parallelschaltung und eine Reihenschaltung von Sensoren kann auch kombiniert werden, sodass in einem parallelen Zweig mehrere Sensoren in Reihe geschaltet sind.

Hierbei ist es denkbar, dass das Gerät zwei oder mehr Mikrofluidaktuatoren aufweist, wobei jeweils ein Mikrofluidaktuator mit einem Sensor in Fluidverbindung steht. Unabhängig davon, ob die mehreren Sensoren in einer Reihenschaltung oder in einer Parallelschaltung miteinander verbunden sind, ist jedem einzelnen Sensor vorzugsweise jeweils ein eigener Mikrofluidaktuator zugeordnet. Somit kann jeder Sensor unabhängig voneinander betrieben werden. Dies erhöht die Betriebssicherheit z.B. beim Ausfall eines Mikrofluidaktuators, sodass die anderen noch betriebsfähigen Mikrofluidaktuatoren weiter ihren Dienst verrichten können.

Es ist vorstellbar, dass zwischen der Öffnung und dem Sensor ein luftdurchlässiges Filterelement angeordnet ist, das ausgebildet ist, um in dem angesaugten Fluid kondensierte Flüssigkeit aufzufangen. Hierbei kann es sich beispielsweise um einen Aktivkohlefilter, einen hydrophoben Filter, einen hydrostatisch geladenen Filter oder einen Teflonfilter handeln. Das luftdurchlässige Filterelement kann eine luftdurchlässige Membran sein, die kondensierte Flüssigkeit auffängt, z.B. eine hydrophobe (z.B. teflonbeschichtete) Filtermembran mit kleiner Porengröße und großem "bubble point" (Blaspunkt), um den Sensor vor Flüssigkeit zu schützen.

Alternativ oder zusätzlich ist es denkbar, dass zwischen dem Sensor und dem Mikrofluidaktuator ein luftdurchlässiges Filterelement angeordnet ist, das ausgebildet ist, um in dem angesaugten Fluid kondensierte Flüssigkeit aufzufangen. Hierbei kann es sich ebenfalls beispielsweise um einen Aktivkohlefilter, einen hydrophoben Filter, einen hydrostatisch geladenen Filter oder einen Teflonfilter handeln. Das luftdurchlässige Filterelement kann eine luftdurchlässige Membran sein, die kondensierte Flüssigkeit auffängt, z.B. eine hydrophobe (z.B. teflonbeschichtete) Filtermembran mit kleiner Porengröße und großem "bubble point", um den Sensor vor Flüssigkeit zu schützen.

Gemäß Ausführungsformen ist das Gerät ein Mobilgerät. Gemäß denkbaren Ausführungsformen kann das Mobilgerät ein Mobiltelefon oder ein Smartphone oder ein Wearable oder ein mobiler Computer sein. Wearables sind am Körper tragbare Mobilgeräte, insbesondere Computer, z.B. in Form von Uhren, Stirnbändern und dergleichen. Mobile Computer sind auch als Laptops, Notebooks oder Netbooks bekannt.

Ein weiterer Vorteil der Erfindung ist, dass mit diesem "Einhubaktor" die zu messende Luft (im Vergleich zu Mikropumpen) extrem schnell zum Sensor gesaugt werden kann: Wenn die Spannung sehr schnell angelegt wird (z.B. innerhalb einer oder einiger Millisekunden), dann bewegt sich auch der Aktor in dieser Zeitkonstante. Der erzeugte Unter- bzw. Überdruck "treibt" dann das Fluid in den Sensorkanal. "Bremsend" wirkt dann nur noch die viskose Reibung der Luft am Kanal oder am Filterelement. Durch entsprechende Auslegung ist es möglich, in weniger als einer Sekunde, bzw. in weniger als 100 Millisekunden, die Umgebungsluft an den Sensor zu befördern.

Mit Mikromembranpumpen wäre diese Geschwindigkeit nur sehr schwer zu schaffen: wollte man ein Totvolumen von z.B. 5 mm³ innerhalb von 0,1 Sekunden fördern, dann würde man eine Pumprate von 50 mm³/s oder 3 ml/min benötigen. Derartige Förderraten sind aber z.B. mit Silizium Mikropumpen nur dann realistisch, wenn die Chipgröße und damit das Hubvolumen groß ist. Damit wären diese Mikropumpen aber teuer und unwirtschaftlich.

Aus der Piezotechnologie ist bekannt, dass ein Piezo Membranwandler nicht zu schnell angesteuert werden soll, um die Lebensdauer nicht herabzusetzen. Dabei muss beachtet werden, dass die Spannungsflanke nicht so schnell ist, dass Moden über der mechanischen Eigenfrequenz angeregt werden. Ansonsten entstehen hohe mechanische Belastungen, und es könnte unterkritisches Risswachstum induziert werden, das den Piezoeffekt verringert und auch zu Brüchen der Piezokeramik führen kann.

Die Eigenresonanz hängt ab von der Geometrie und dem Elastizitätsmodul von Membran und Piezo. Bei "normalen" Mikropumpen liegen die Eigenfrequenzen in der Größenordnung 10...30 kHz. Bei dieser Idee muss das Hubvolumen groß sein, während der Blockierdruck nicht zu groß sein soll. Die Eigenfrequenzen können daher deutlich tiefer liegen, bis hinab zu 1 kHz oder darunter. Um eine lange Lebensdauer zu gewährleisten, muss darauf geachtet werden, die Anstiegsflanke der Antriebsspannung deutlich langsamer zu wählen, z.B. 10 Millisekunden oder darüber. Diese Anstiegszeit schützt den Mikroaktor vor Rissen, ist aber noch genügend schnell für eine Ansprechzeit unter einer Sekunde.

Weiterhin könnte man mit diesem Mikroaktor auch andere Betriebsmodi wählen, indem das Spannungssignal geeignet moduliert wird. Zum Beispiel kann die Strömungsgeschwindigkeit, mit der das zu messende Gas am Sensorelement vorbeiströmt, durch die Aktorgeschwindigkeit einfach variiert werden. Oder es können gepulste Gasportionen erzeugt werden. Oder das Gas kann in einem ersten Schritt zu einem ersten Sensor, in einem zweiten Schritt zu einem dahinter liegenden Sensor gefördert werden. Je nach Sensorprinzip kann dies vorteilhaft sein.

Ausführungsbeispiele der Erfindung sind in der Zeichnung dargestellt und werden nachstehend erläutert. Es zeigen:
- Fig. 1: eine Draufsicht auf eine Ausführungsform eines erfindungsgemäßen Mobilgeräts,
- Fig. 2: eine Schnittansicht einer Ausführungsform eines erfindungsgemäßen Mobilgeräts entlang der in Figur 1 gezeigten Schnittlinie II-II,
- Fig. 3: eine Schnittansicht einer Ausführungsform eines erfindungsgemäßen Mobilgeräts,
- Fig. 4: eine Schnittansicht einer Ausführungsform eines erfindungsgemäßen Mobilgeräts mit einer Reihenschaltung mehrerer Sensoren,
- Fig. 5: eine Draufsicht auf eine Ausführungsform eines erfindungsgemäßes Mobilgeräts mit einer Parallelschaltung mehrerer Sensoren, und
- Fig. 6: eine Draufsicht auf eine weitere Ausführungsform eines erfindungsgemäßen Mobilgeräts mit einer Parallelschaltung mehrerer Sensoren.

Bezugnehmend auf die Figuren werden im Folgenden Ausführungsbeispiele anhand von Mobilgeräten beschrieben. Bei Ausführungsformen kann das Gerät ein stationäres Gerät sein, wie z.B. ein Sensorknoten oder dergleichen, so dass die jeweiligen Ausführungen auch für stationäre Geräte gelten. Ein stationärer Sensorknoten kann beispielsweise autark batteriebetrieben arbeiten und Sensordaten aufnehmen und kommunizieren.

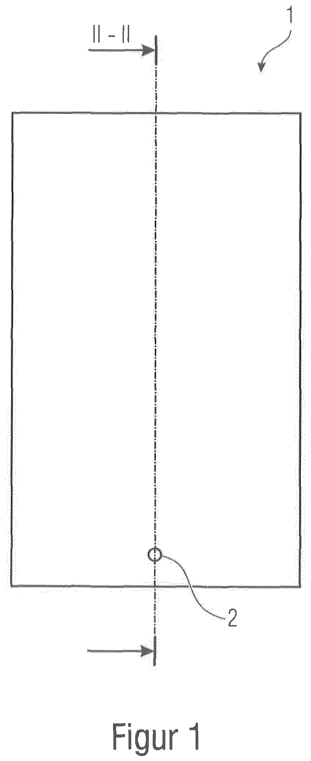

Figur 1 zeigt eine Ausführungsform eines erfindungsgemäßen Mobilgeräts 1 mit einer Öffnung 2. Die Öffnung 2 ist eine Gehäuseöffnung, die eine Fluidverbindung zwischen einem Fluidkanal in dem Mobilgerät und Umgebungsluft definiert.

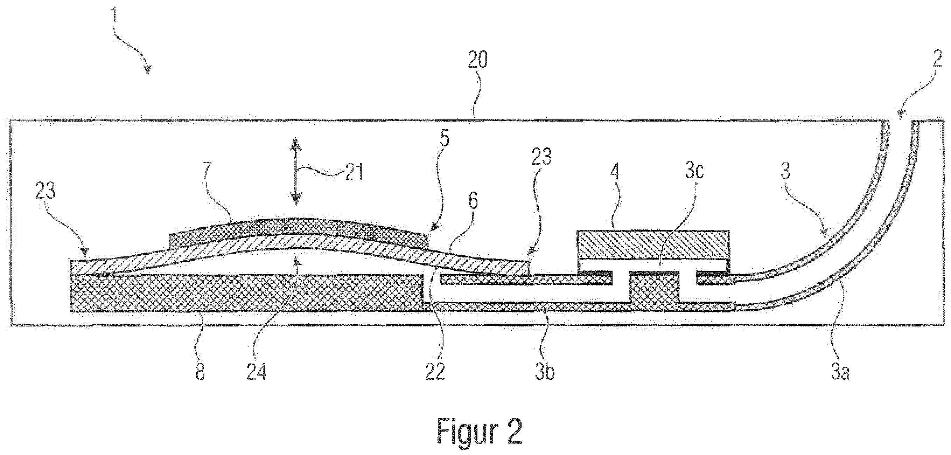

Figur 2 ist eine Schnittansicht durch das Mobilgerät 1 entlang der in Figur 1 gezeigten Schnittlinie II-II. In Figur 2 ist das Gehäuse 20 des Mobilgeräts 1 zu erkennen. Das Gehäuse 20 weist die Öffnung 2 auf.

In dem Mobilgerät 1 ist die zuvor erwähnte Fluidverbindung 3 angeordnet. Ein Sensor 4 ist mit dem Fluidkanal 3 gekoppelt. Der Sensor 4 ist ausgebildet, um zumindest einen Bestandteil der Umgebungsluft zu erfassen.

In dem Mobilgerät 1 ist außerdem ein Mikrofluidaktuator 5 angeordnet. Der Mikrofluidaktuator 5 ist ebenfalls mit dem Fluidkanal 3 gekoppelt. In dem Fluidkanal 3 ist der Mikrofluidaktuator 5 dem Sensor 4 nachgeschaltet. In anderen Worten ist der Sensor 4 mit dem Fluidkanal 3 fluidisch gekoppelt und zwischen der Öffnung 2 und dem Mikrofluidaktuator 5 angeordnet.

Somit weist der Fluidkanal 3 einen ersten Abschnitt 3a auf, der sich zwischen der Öffnung 2 und dem Sensor 4 erstreckt, sowie einen zweiten Abschnitt 3b, der sich zwischen dem Sensor 4 und dem Mikrofluidaktuator 5 erstreckt.

Der Mikrofluidaktuator 5 ist ausgebildet, um im Saughub Fluid durch den Fluidkanal 3anzusaugen und in Richtung des Sensors 4 zu befördern. Außerdem ist der Mikrofluidaktuator 5 ausgebildet, um im Druckhub das angesaugte und in dem Fluidkanal 3 befindliche Fluid durch den Fluidkanal 3 wieder zurück in Richtung der Öffnung 2 zu befördern.

Der Sensor 4 ist von der Öffnung 2 beabstandet angeordnet. Im Vergleich zu bekannten Lösungsansätzen, die vorsehen, den Sensor 4 möglichst nahe an der Öffnung 2 zu platzieren, um die Wege der Fluidströmung gering zu halten, bietet eine erfindungsgemäße beabstandete Anordnung des Sensors 4 die Möglichkeit, den Sensor 4 an nahezu beliebigen Stellen in dem Mobilgerät 1 anzuordnen. Dies ist insbesondere bei heutzutage immer flacher bauenden Mobilgeräten 1 wünschenswert, da diese nicht uneingeschränkt Platz zum Einbau von Sensorik bieten.

Erfindungsgemäß ist das Volumen des ersten Abschnitts 3a des Fluidkanals 3 zwischen dem Sensor 4 und der Öffnung 2 genauso groß oder kleiner als das Hubvolumen, das der Mikrofluidaktuator 5 mit einem einzelnen Saughub fördern kann.

Der Fluidkanal 3 erstreckt sich zwischen der Öffnung 2 und dem Mikrofluidaktuator 5. Der Fluidkanal 3, der mit dem Fluidkanal 3 gekoppelte Sensor 4 und der Mikrofluidaktuator 5 bilden zusammen eine Anordnung, die gegenüber dem Innenraum des Mobilgeräts 1 abgedichtet ist. Das heißt, der Fluidkanal 3 sowie der Sensor 4 und der Mikrofluidaktuator 5 sind dicht, insbesondere luftdicht ausgeführt.

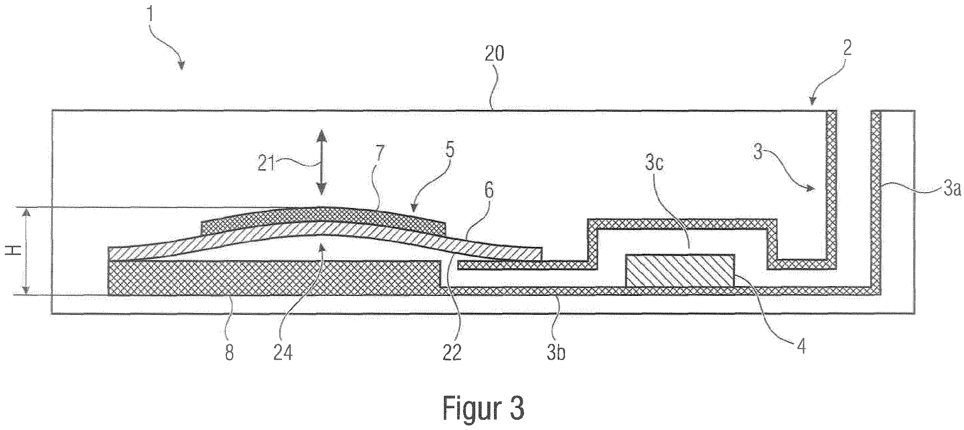

In den in den Figuren 2 und 3 abgebildeten Ausführungsbeispielen ist der Mikrofluidaktuator 5 als ein Membranaktuator ausgebildet. Der Mikrofluidaktuator 5 weist eine auslenkbare Membran 6 auf. Die Membran 6 kann piezoelektrisch, elektromagnetisch, elektrostatisch, mittels elektroaktiven Polymeraktoren oder mittels eines Elements aufweisend eine Formgedächtnislegierung betätigt werden.

Ein entsprechend ausgebildetes Betätigungsmittel 7 ist zum Zwecke der Auslenkung der Membran 6 an dieser angeordnet. Wie in den Figuren zu erkennen ist, ist die Membran 6 mit einer Seite 22 an einem Träger 8 angeordnet. Der Träger 8 kann beispielsweise als ein Siliziumchip oder Metall- oder Polymerkörper ausgebildet sein.

Genauer gesagt, ist die Membran 6 an deren lateral außenliegenden Abschnitten 23 an dem Träger 8 angeordnet. Die Membran 6 kann in Draufsicht eine runde oder eine eckige, insbesondere eine hexagonale, Form aufweisen. Die Membran 6 kann mittels Befestigungsmittel, z.B. mittels eines geeigneten Klebstoffs, auf dem Träger 8 befestigt bzw. fixiert werden.

Die Membran 6 ist in den in den Figuren abgebildeten Ausführungsbeispielen unter mechanischer Vorspannung auf dem Träger 8 angeordnet. Das heißt, die Membran 6 weist eine durch die Vorspannung bedingte konkave (von dem Träger 8 weg gerichtete) Wölbung auf. Dadurch bildet sich zwischen dem Träger 8 und der Membran 6 im Bereich dieser Wölbung eine Kavität 24 aus. Das Volumen dieser Kavität 24 bestimmt im Wesentlichen das Hubvolumen des Mikrofluidaktuators 5.

Wie in den Figuren 2 und 3 mit dem Doppelpfeil 21 angedeutet ist, kann sich der Mikrofluidaktuator 5 im Wesentlichen in zwei Richtungen bewegen. In einer unbetätigten Ruheposition befindet sich der Mikrofluidaktuator 5 in der abgebildeten Stellung, in der die Membran 6 vorgespannt ist und sich die zuvor erwähnte Kavität 24 ausbildet. In dieser Ruheposition ist die Membran 6 also von dem Träger 8 beabstandet.

Das Betätigungsmittel 7 kann nun die Membran 6 betätigen. Beispielsweise kann das Betätigungsmittel 7 ein Piezoelement sein, das beim Anlegen einer Spannung die Membran 6 nach unten, d.h. in Richtung zu dem Träger 8 hin, auslenkt. Bei der Betätigung der Membran 6 bewegt sich diese also zu dem Träger 8 hin, bis die Membran 6 mit ihrer Unterseite 22 in Kontakt mit dem Träger 8 kommt.

Dabei verdrängt der Mikrofluidaktuator 5 das Volumen des zu diesem Zeitpunkt in der Kavität 24 befindlichen Fluids. Dieses Fluidvolumen wird dann durch den Fluidkanal 3 befördert und tritt aus der Öffnung 2 in die Umgebung aus. Der Mikrofluidaktuator 5 hat in diesem Fall also einen Druckhub ausgeführt und das ausgestoßene Fluidvolumen entspricht im Wesentlichen dem Hubvolumen des Mikrofluidaktuators 5.

Das Betätigungsmittel 7 kann nun die Membran 6 wieder in die entgegengesetzte Richtung, d.h. in Richtung von dem Träger 8 weg, bewegen. Im Falle einer vorgespannten Membran 6 ist es bereits ausreichend, wenn das Betätigungsmittel 7 keine Betätigungskraft mehr auf die Membran 6 ausübt. Die Membran 6 kehrt dann aufgrund der mechanischen Vorspannung wieder in ihre ursprüngliche Ausgangsposition zurück.

In diesem Fall bewegt sich also die Membran 6 von dem Träger 8 weg und vergrößert somit wieder die sich zwischen der Membranunterseite 22 und dem Träger 8 ausbildende Kavität 24. Es bildet sich ein Unterdruck in der fluiddichten Anordnung (Mikrofluidaktuator 5, Sensor 4, Fluidkanal 3) aus, und Fluid wird aus der Umgebung durch die Öffnung 2 angesaugt. Der Mikrofluidaktuator 5 führt in diesem Fall also einen Saughub aus.

Der Fluidkanal 3, d.h. der vordere Abschnitt 3a, der hintere Abschnitt 3b und der den Sensor 4 umströmende Abschnitt 3c des Fluidkanals 3, weisen ein bestimmtes Volumen auf. Dieses Volumen wird auch als Gesamttotvolumen bezeichnet.

Erfindungsgemäß ist das Hubvolumen des Mikrofluidaktuators 5 mindestens genauso groß wie das Gesamttotvolumen. Es kann jedoch bereits ausreichend sein, wenn das Hubvolumen des Mikrofluidaktuators 5 mindestens genauso groß ist wie das Volumen des vorderen Fluidkanalabschnitts 3a. Ein Saughub des Mikrofluidaktuators 5 fördert somit gerade soviel Volumen durch den vorderen Fluidkanalabschnitt 3a, dass ein an der Öffnung 2 angesaugtes Fluid gerade den Sensor 4 erreicht.

Um das Umströmen des Sensors 4 mit angesaugtem Fluid sicherzustellen, sehen Ausführungsformen der Erfindung vor, dass das Hubvolumen des Mikrofluidaktuators 5 um mindestens das 2,5-fache größer ist als das Volumen des Fluidkanals 3 zwischen dem Sensor 4 und der Öffnung 2, d.h. als der vordere Fluidkanalabschnitt 3a.

In der in Figur 2 abgebildeten Ausführungsform ist der Sensor 4 fluidisch mit dem Fluidkanal 3 gekoppelt, indem der Sensor 4 an dem Fluidkanal 3 angeordnet ist. In der in Figur 3 abgebildeten Ausführungsform ist der Sensor 4 fluidisch mit dem Fluidkanal 3 gekoppelt, indem der Sensor 4 nicht an sondern in dem Fluidkanal 3 angeordnet ist.

Wie in Figur 3 zu erkennen ist, weist der Mikrofluidaktuator 5 eine Höhe H auf, die sich zwischen der Unterseite des Trägers 8 und der Oberseite der Membran 6 bzw. des an der Membran 6 angeordneten Betätigungsmittels 7 erstreckt. Gemäß vorteilhaften Ausführungsformen weist der Mikrofluidaktuator 5 eine Höhe H von 0,50 mm oder weniger auf.

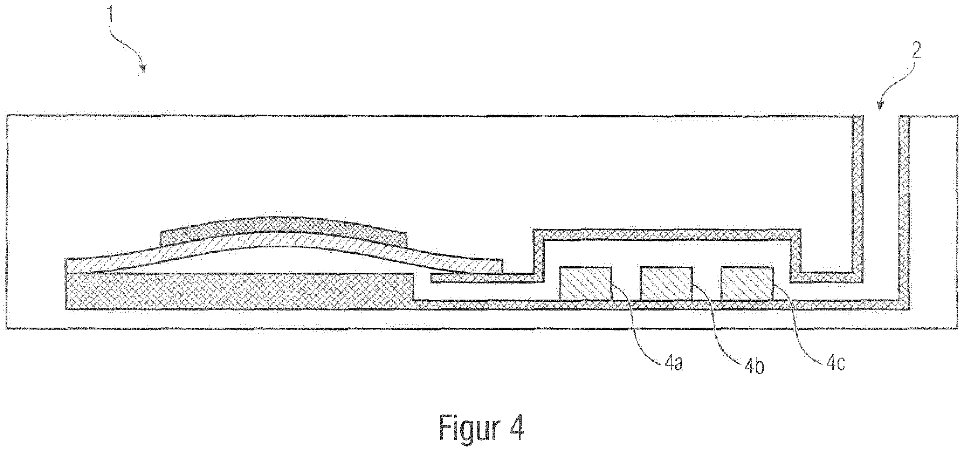

Figur 4 zeigt eine weitere Ausführungsform eines erfindungsgemäßen Mobilgeräts 1. Hier weist das Mobilgerät 1 drei Sensoren 4a, 4b, 4c auf, die in Strömungsrichtung (sowohl in Ansaugrichtung als auch in Ausstoßrichtung) nacheinander in Reihe angeordnet sind.

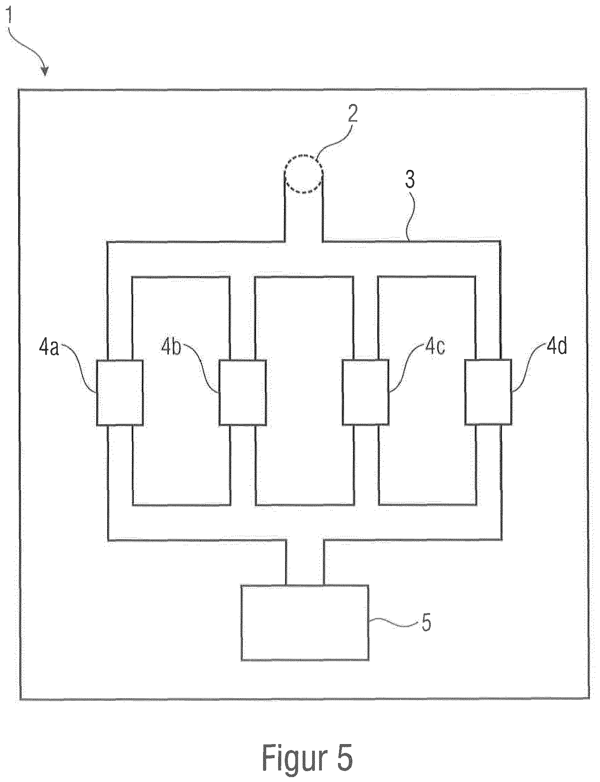

Figur 5 zeigt ein weiteres Ausführungsbeispiel, wobei das Mobilgerät 1 vier Sensoren 4a, 4b, 4c, 4d aufweist, die in Strömungsrichtung parallel zueinander angeordnet sind. Das Mobilgerät 1 weist in diesem Ausführungsbeispiel einen Mikrofluidaktuator 5 auf, der ausgebildet ist, um die Parallelschaltung aus mehreren Sensoren 4a bis 4d gemeinsam mit angesaugter Umgebungsluft zu versorgen.

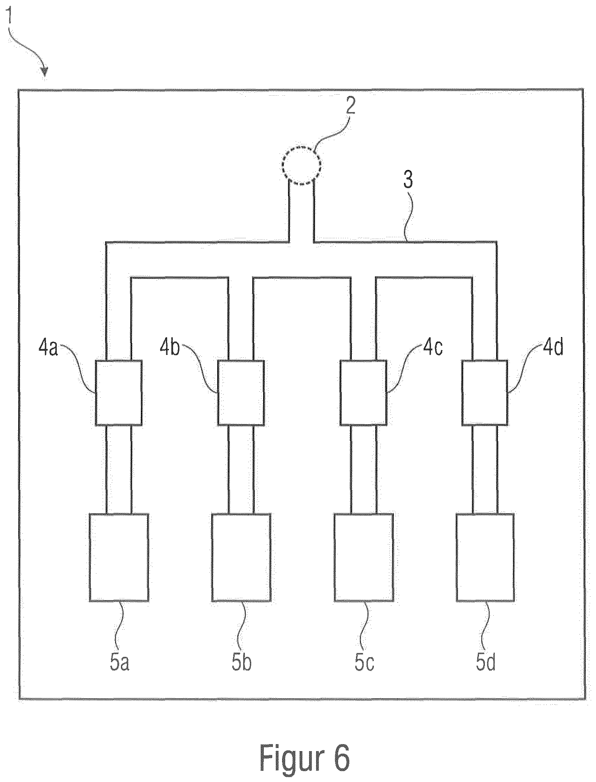

Figur 6 zeigt ein weiteres Ausführungsbeispiel, wobei das Mobilgerät 1 vier Mikrofluidaktuatoren 5a, 5b, 5c, 5d sowie vier Sensoren 4a, 4b, 4c, 4d aufweist. Hier steht jeder Mikrofluidaktuator 5a, 5b, 5c, 5d mit jeweils einem Sensor 4a, 4b, 4c, 4d in Fluidverbindung. Beispielsweise kann jeder der vier Sensoren 4a bis 4d einen bestimmten Umgebungsluftparameter analysieren. Somit können die Sensoren 4a, bis 4d bedarfsgerecht arbeiten, indem der jeweils zugehörige Mikrofluidaktuator 5a bis 5d separat angesteuert wird.

Nachdem die Ausführungsform des erfindungsgemäßen Mobilgeräts 1 strukturell beschrieben wurde, soll im Folgenden kurz die Funktionsweise erläutert werden.

Der Sensor 4 kann beispielsweise ein Sensor zum Messen von Kohlenstoffmonoxid (CO), Kohlendioxid (CO₂), Stickstoff (N), Distickstoffmonoxid (N₂O), flüchtigen organischen Verbindungen (VOC), Luftfeuchtigkeit oder Feinstaub sein. Hierfür muss der Sensor 4 mit Umgebungsluft umströmt werden.

Das Mobilgerät 1 kann beispielsweise ein Smartphone mit einer bereits vorhandenen Mikrofonöffnung 2 sein. Durch diese Mikrofonöffnung 2 kann die Umgebungsluft angesaugt werden. Hierfür ist an der Mikrofonöffnung 2 eine Fluidleitung 3 angeordnet. Diese Fluidleitung 3 führt zu dem Sensor 4.

Eine Diffusion der Umgebungsluft von der Öffnung 2 bis hin zu dem Sensor 4 dauert verhältnismäßig lange. Deshalb ist in der Erfindung der Mikrofluidaktuator 5 vorgesehen, der die Umgebungsluft durch die Öffnung 2 ansaugt. Die angesaugte Umgebungsluft strömt somit wesentlich schneller zu dem Sensor 4, der wiederum entsprechend schneller Signale generieren kann. Die bisher bekannten Lösungen, die nach dem Diffusionsprinzip funktionieren, werden nun also durch Konvektion ersetzt.

Der Mikrofluidaktuator 5 führt also einen Saughub aus, in dem er die Umgebungsluft durch die Öffnung 2 ansaugt. Erfindungsgemäß ist das Volumen des Fluidkanals 3 kleiner als das Hubvolumen des Mikrofluidaktuators 5. Somit strömt die durch die Öffnung 2 angesaugte Umgebungsluft durch den vorderen Fluidkanalabschnitt 3a, durch den dem Sensor 4 benachbarten Abschnitt 3c und durch den hinteren Fluidkanalabschnitt 3b in die Kavität 24 des Mikrofluidaktuators 5. Die angesaugte Umgebungsluft umströmt somit den Sensor 4.

Die angesaugte Umgebungsluft wird anschließend wieder von dem Mikrofluidaktuator 5 durch die Öffnung 2 ausgestoßen. Hierzu führt der Mikrofluidaktuator 5 einen Druckhub aus, in dem die angesaugte Luft in umgekehrter Richtung wieder durch den Fluidkanal 3 strömt und durch die Öffnung 2 an die Umgebung abgegeben wird.

Die Erfindung löst somit das Problem im Stand der Technik, bei dem eine Mikropumpe Luft ansaugt und die angesaugte Luft anschließend wieder ausstößt und zwar in das Mobilgerät hinein. Bei dem erfindungsgemäßen Mobilgerät 1 hingegen saugt der Mikrofluidaktuator 5 ein Fluidvolumen durch den Fluidkanal 3 an und stößt das angesaugte Fluidvolumen auch wieder über diesen Fluidkanal 3 aus. Der Mikrofluidaktuator 5 schiebt sozusagen ein Fluidvolumen in dem Fluidkanal 3 hin und her.

Nachfolgend sollen Ausführungsformen der Erfindung noch einmal in anderen Worten zusammengefasst werden.

Ein Bestandteil dieser Offenbarung ist ein bauflacher Membran-Aktuator, der zyklisch ein Luftvolumen (vor- und zurück) verschieben kann, und an dem ein (oder mehrere) Sensoren und eine Fluidleitung angeschlossen sind.

Das Totvolumen im Schlauch sowie im Fluidadapter bis zum Sensor soll dabei kleiner sein als das (Luft-)volumen, das der Aktuator verschieben kann.

Der Aktuator kann z.B.
- Piezoelektrisch
   ∘ Monomorph Biegewandler
   ▪ Aufgeklebte PZT Keramik
   ▪ Dünnschicht (AIN, Zinkoxid ...)
   ▪ Dickschicht PZT
- elektromagnetisch
- elektrostatisch
- durch elektroaktive Polymeraktoren
- termisch, beispielsweise durch eine Formgedächtnislegierung,
- mit dem Motor für den Vibrationsalarm als Antrieb für die Membran etc. angetrieben werden.

Eine denkbare Ausführungsform ist ein Piezo-Membranwandler 5. Dieser kann sehr flach gebaut werden (Voraussetzung für Mobilfunkgeräte - Bauhöhe mit Träger kleiner 0,5 mm), und trotzdem ein großes Hubvolumen von mehreren mm³ verschieben. Siehe Figur 2 mit einem Piezo-Membranaktuator 5, um frische Luft einem Sensor 4 zuzuführen.

Statt einem Schlauch kann auch ein starres Röhrchen als Fluidkanal 3 verwendet werden.

Alle Fluidverbinder sollten dicht sein, damit die Luft aus der Öffnung 2 angesaugt wird. Es können auch mehrere Sensoren 4 (bevorzugt seriell) angeordnet werden.

Eine parallele Anordnung ist dann sinnvoll, wenn die Strömungswiderstände aller Sensoren 4 in etwa gleich sind.

Es können auch mehrere Aktuatoren 5 verwendet werden, z.B. für jeden Sensor 4 einer.

Gegebenenfalls kann auch eine Doppelmembran genutzt werden, um die Spannung niedrig zu halten.

Die fluidischen Wege sollten so gestaltet werden, dass möglichst wenig Ecken und Toträume vorhanden sind. Dann ist sichergestellt, dass nur wenig Dispersion und Verschleppung stattfindet. Im Idealfall muss das Hubvolumen des Mikroaktuators 5 nur so groß sein wie das Totvolumen (von der Ansaugöffnung 2 bis zum Sensor 4). Real wird das Hubvolumen um einen bestimmten Faktor größer gemacht als das Totvolumen.

Zwischen Ansaugöffnung 2 und Sensor 4, oder zwischen Sensor 4 und Mikrofluidaktuator 5 (z.B. Mikropumpe), kann eine luftdurchlässige Membran angeordnet sein, die keine kondensierte Flüssigkeit durchlässt, z.B. eine hydrophobe (z.B. teflonbeschichtete) Filtermembran mit kleiner Porengröße und großem "bubble point", um den Sensor 4 vor Flüssigkeit zu schützen. Diese Membran hat, je nach Auslegung, einen Strömungswiderstand zu überwinden. Außerdem kann es bei der Zuleitung Druckabfälle geben.

Daher sollte der Mikroaktuator 5 auch
- einen bestimmten Blockierdruck erzeugen können,
- und auch ein bestimmtes Kompressionsverhältnis zwischen Hubvolumen und Gesamttotvolumen (Totvolumen von Fluidleitung 3, Sensor 4, Fluidadapter 5 und Aktuatorkammer 24) erfüllen.

Um das sicherzustellen, wird der Mikroaktuator 5 vorzugsweise durch die Vorspannungsmethode (patentiert durch Fraunhofer EMFT) aufgebracht, um das Totvolumen der Aktuatorkammer 24 zu minimieren.

Vor dem Ansaugen der Luftprobe wird der Membranaktuator 5 betätigt (z.B. durch Anlegen einer positiven Spannung), und fährt in seine untere Position. Das Totvolumen ist nun minimal.

Danach wird der Aktuator 5 in seine obere Position bewegt (z.B. durch Ausschalten der Spannung, bzw. Anlegen einer geeigneten negativen Spannung). Dabei entsteht in der Aktuatorkammer 24 ein Unterdruck, es wird Umgebungsluft rasch angesaugt, und dem Sensor 4 zugeführt, damit kann die Messung der Luftparameter stattfinden.

### Zahlenbeispiel:

### Aktuator 1 (Durchmesser 9,6 mm)

- Baugröße von 10x10x0,5 mm³,
- einem Membrandurchmesser von 9,6 mm
- Membrandicke 30 µm
- Piezodicke 60 µm
- d31: 250 m/V
- Antriebsspannung: +90 / -24 V

### Folgende Hubdaten:

- Hubvolumen: 2,35 mm³
- Blockierdruck 14 kPa

### Aktuator 2 (Durchmesser 14,6 mm)

- Baugröße von 15x15x0,5 mm³,
- einem Membrandurchmesser von 14,6 mm
- Membrandicke 40 µm
- Piezodicke 80 µm
- d31: 250 m/V
- Antriebsspannung : +120 / -32 V

### Folgende Hubdaten:

- Hubvolumen: 9,4 mm³
- Blockierdruck 11 kPa

### Totvolumen von Öffnung bis Sensor:

### Schlauch bzw. Röhrchen

- Länge 10 mm
- Innendurchmesser 0,2 mm
- Totvolumen = I r² π = 10 mm x (0,1 mm)² x 3,14 = 0,32 mm³

### Fluidadapter

- Länge 2 mm
- Innendurchmesser 0,2 mm
- Totvolumen = I r² π = 2 mm x (0,1 mm)² x 3,14 = 0,06 mm³

### Sensorgehäuse

- Länge:1,5x 1,5 mm²
- Höhe 0,2 mm
- Totvolumen = 0,45 mm³

### Gesamttotvolumen

- 0,83 mm³

D.h. das Hubvolumen ist bei Aktuator 1 um einen Faktor 2,8 größer, bei Aktuator 2 um einen Faktor 11,3 als das Totvolumen, es kommt bei jedem Hub sicher Frischluft an den Sensor.

Gegebenenfalls kann es sinnvoll sein, dickere Piezokeramiken zu verwenden, da bei Verwendung von so dünnen Keramiken mit so großen lateralen Abmessungen die mechanischen Belastungen in der Piezokeramik sehr groß werden, und es durch zu hohe Zugspannungen in der Piezokeramik zu Brüchen kommen kann.

Ein Ausführungsbeispiel hier wäre:

### Aktuator 3 (Durchmesser 9,6 mm)

- Baugröße von 10x10x0,5 mm³,
- einem Membrandurchmesser von 9,6 mm
- Membrandicke 30 µm
- Piezodicke 150 µm
- d31: 250 m/V
- Antriebsspannung: +225 / -60 V

### Folgende Hubdaten:

- Hubvolumen: 1,3 mm³
- Blockierdruck 29 kPa

### Aktuator 4 (Durchmesser 14,6 mm)

- Baugröße von 15x15x0,5 mm³,
- einem Membrandurchmesser von 14,6 mm
- Membrandicke 50 µm
- Piezodicke 150 µm
- d31: 250 m/V
- Antriebsspannung : +225 / -60 V

### Folgende Hubdaten:

- Hubvolumen: 6,3 mm³
- Blockierdruck 23 kPa

Die beiden letzten Aktoren haben weniger Hubvolumen (aber bei geeignetem Design der Zuleitungen immer noch genug), aber sie sind bezüglich mechanischem Stress "konservativer" ausgelegt.

Mit einem Doppelhubaktor kann das Hubvolumen jeweils verdoppelt werden

## Patentansprüche

1. Gerät (1) mit
einem Gehäuse mit einer Gehäuseöffnung (2), die eine Fluidverbindung zwischen einem Fluidkanal (3) in dem Gerät (1) und Umgebungsluft definiert,
einem mit dem Fluidkanal (3) gekoppelten und in dem Gerät (1) angeordneten Sensor (4), der ausgebildet ist, um zumindest einen Bestandteil der Umgebungsluft zu erfassen,
einem dem Sensor (4) nachgeschalteten und in dem Gerät (1) angeordneten Mikrofluidaktuator (5), der ausgebildet ist, um im Saughub Fluid durch den Fluidkanal (3) anzusaugen und in Richtung des Sensors (4) zu befördern und im Druckhub das angesaugte Fluid durch ebendiesen Fluidkanal (3) wieder zurück in Richtung der Gehäuseöffnung (2) zu befördern,
wobei der Sensor (4) von der Gehäuseöffnung (2) beabstandet angeordnet ist, und das Volumen des Fluidkanals (3) zwischen dem Sensor (4) und der Gehäuseöffnung (2) genauso groß oder kleiner ist als das Hubvolumen, das der Mikrofluidaktuator (5) mit einem einzelnen Saughub fördern kann,
**dadurch gekennzeichnet, dass**
das Hubvolumen des Mikrofluidaktuators (5) um mindestens das 2,5-fache größer ist als das Volumen eines Fluidkanalabschnitts (3a) zwischen dem Sensor (4) und der Gehäuseöffnung (2), und
dadurch dass
der Mikrofluidaktuator (5) ein Membranaktuator mit einem Träger (8) und einer daran angeordneten auslenkbaren Membran (6) ist, und die Membran (6) mechanisch vorgespannt ist, sodass die Membran (6) in einer unbetätigten Ruheposition von dem Träger (8) beabstandet ist und sich bei Betätigung zu dem Träger (8) hin bewegt.

2. Gerät (1) nach Anspruch 1, wobei sich der Fluidkanal (3) zwischen der Gehäuseöffnung (2) und dem Mikrofluidaktuator (5) erstreckt, und der Fluidkanal (3), der mit dem Fluidkanal (3) gekoppelte Sensor (4) und der Mikrofluidaktuator (5) zusammen eine fluiddichte Anordnung bilden, die gegenüber dem Innenraum des Geräts (1) abgedichtet ist.

3. Gerät (1) nach einem der vorhergehenden Ansprüche, wobei das Hubvolumen des Mikrofluidaktuators (5) um mindestens das 10-fache größer ist als das Volumen des Fluidkanalabschnitts (3a) zwischen dem Sensor (4) und der Gehäuseöffnung (2).

4. Gerät (1) nach einem der vorhergehenden Ansprüche, wobei der Sensor (4) ausgebildet ist, um mindestens einen Umgebungsluftbestandteil aus der Gruppe von Kohlenstoffmonoxid (CO), Kohlendioxid (CO₂), Stickstoff (N₂), Distickstoffmonoxid (N₂O), flüchtige organische Verbindungen (VOC), Luftfeuchtigkeit und Feinstaub zu erfassen.

5. Gerät (1) nach einem der vorhergehenden Ansprüche, wobei die Membran (6) piezoelektrisch oder elektromagnetisch oder elektrostatisch oder mittels elektroaktiven Polymeraktoren oder mittels einem Element (7) aufweisend eine Formgedächtnislegierung betätigbar ist.

6. Gerät (1) nach einem der vorhergehenden Ansprüche, wobei der Mikrofluidaktuator (5) ein Membranaktuator mit einer auslenkbaren Doppelmembran (6) ist.

7. Gerät (1) nach einem der vorhergehenden Ansprüche, wobei die Membran (6) einschließlich des Trägers (8) eine Höhe von 0,50 mm oder weniger aufweist.

8. Gerät (1) nach einem der vorhergehenden Ansprüche, wobei das Gerät (1) zwei oder mehr Sensoren (4a, 4b, 4c, 4d) aufweist, die in Strömungsrichtung nacheinander in Reihe angeordnet sind, oder wobei das Gerät (1) zwei oder mehr Sensoren (4a, 4b, 4c, 4d) aufweist, die in Strömungsrichtung parallel zueinander angeordnet sind.

9. Gerät (1) nach Anspruch 8, wobei das Gerät (1) zwei oder mehr Mikrofluidaktuatoren (5a, 5b, 5c, 5d) aufweist, wobei jeweils ein Mikrofluidaktuator (5a, 5b, 5c, 5d) mit einem Sensor (4a, 4b, 4c, 4d) in Fluidverbindung steht.

10. Gerät (1) nach einem der vorhergehenden Ansprüche, wobei zwischen der Gehäuseöffnung (2) und dem Sensor (4) und/oder zwischen dem Sensor (4) und dem Mikrofluidaktuator (5), ein luftdurchlässiges Filterelement angeordnet ist, das ausgebildet ist, um in dem angesaugten Fluid kondensierte Flüssigkeit aufzufangen.

11. Gerät (1) nach einem der vorhergehenden Ansprüche, wobei das Gerät ein Mobilgerät ist.

12. Gerät (1) nach Anspruch 11, wobei das Mobilgerät (1) ein Mobiltelefon ist und die in dem Mobiltelefon vorgesehene Gehäuseöffnung (2) eine Mikrofonöffnung ist.

13. Gerät (1) nach Anspruch 11 oder 12, wobei das Mobilgerät (1) ein Mobiltelefon mit einem Vibrationsalarmmotor ist und der Mikrofluidaktuator (5) ein Membranaktuator mit einer auslenkbaren Membran (6) ist, wobei die Membran (6) mittels des Vibrationsalarmmotors betätigbar ist.

14. Gerät (1) nach Anspruch 13, wobei das Mobilgerät (1) ein Mobiltelefon oder ein Smartphone oder ein Wearable oder ein mobiler Computer ist.

15. Gerät nach einem der Ansprüche 1 bis 10, wobei das Gerät ein stationäres Gerät ist.

## Claims

1. Device (1) comprising:
a housing with a housing opening (2) defining a fluid connection between a fluid channel (3) in the device (1) and ambient air,
a sensor (4) coupled to the fluid channel (3) and disposed in the housing (1), configured to sense at least one component of the ambient air,
a micro fluid actuator (5) arranged downstream of the sensor (4) and disposed in the housing (1), configured, in the suction stroke, to suck in fluid through the fluid channel (3) and to transport the same towards the sensor (4), and, in the pressure stroke, to transport the sucked-in fluid through said fluid channel (3) back towards the housing opening (2),
the sensor (4) being arranged spaced apart from the housing opening (2), and the volume of the fluid channel (3) between the sensor (4) and the housing opening (2) being equal to or smaller than the stroke volume which the micro fluid actuator (5) may convey with a single suction stroke,
**characterized in that**
the stroke volume of the micro fluid actuator (5) is at least 2.5 times larger than the volume of a fluid channel portion (3a) between the sensor (4) and the housing opening (2), and
**in that** the micro fluid actuator (5) is a membrane actuator having a carrier (8) and a deflectable membrane (6) arranged at the same, and the membrane (6) is mechanically biased so that the membrane (6) is spaced apart from the carrier (8) in an unoperated idle position and, upon operation, moves towards the carrier (8).

2. Device (1) according to claim 1, wherein the fluid channel (3) extends between the housing opening (2) and the micro fluid actuator (5), and the fluid channel (3), the sensor (4) coupled to the fluid channel (3), and the micro fluid actuator (5) together form a fluid-tight arrangement which is sealed against the interior of the device (1).

3. Device (1) according to one of the preceding claims, wherein the stroke volume of the micro fluid actuator (5) is at least 10 times larger than the volume of the fluid channel portion (3a) between the sensor (4) and the housing opening (2).

4. Device (1) according to one of the preceding claims, wherein the sensor (4) is configured to sense at least one ambient air component from the group of carbon monoxide (CO), carbon dioxide (CO₂), nitrogen (N₂), nitrous oxide (N₂O), volatile organic compounds (VOC), humidity and fine dust.

5. Device (1) according to one of the preceding claims, wherein the membrane (6) is operable in a piezoelectric or electromagnetic or electrostatic manner or by means of electroactive polymer actuators or by means of an element (7) comprising a shape memory alloy.

6. Device (1) according to one of the preceding claims, wherein the micro fluid actuator (5) is a membrane actuator having a deflectable double membrane (6).

7. Device (1) according to one of the preceding claims, wherein the membrane (6) including the carrier (8) comprises a height of 0.50 mm or less.

8. Device (1) according to one of the preceding claims, wherein the device (1) comprises two or more sensors (4a, 4b, 4c, 4d) arranged successively in series in the flow direction or wherein the device (1) comprises two or more sensors (4a, 4b, 4c, 4d) arranged in parallel in the flow direction.

9. Device (1) according to claim 8, wherein the device (1) comprises two or more micro fluid actuators (5a, 5b, 5c, 5d), each micro fluid actuator (5a, 5b, 5c, 5d) being in fluid connection with a respective sensor (4a, 4b, 4c, 4d).

10. Device (1) according to one of the preceding claims, wherein an air-permeable filter element is arranged between the housing opening (2) and the sensor (4) and/or between the sensor (4) and the micro fluid actuator (5), configured to collect liquid condensed in the sucked-in fluid.

11. Device (1) according to one of the preceding claims, wherein the device is a mobile device.

12. Device (1) according to claim 11, wherein the mobile device (1) is a mobile telephone, and the housing opening (2) provided in the mobile telephone is a microphone opening.

13. Device (1) according to claim 11 or 12, wherein the mobile device (1) is a mobile telephone having a vibrating alert motor, and the micro fluid actuator (5) is a membrane actuator having a deflectable membrane (6), the membrane (6) being operable by means of the vibrating alert motor.

14. Device (1) according to claim 13, wherein the mobile device (1) is a mobile telephone or a smartphone or a wearable or a mobile computer.

15. Device according to one of claims 1 to 10, wherein the device is a stationary device.

## Revendications

1. Appareil (1) avec
un boîtier avec une ouverture de boîtier (2) qui définit une communication en fluide entre un canal à fluide (3) dans l'appareil (1) et l'air ambiant,
un capteur (4) couplé au canal à fluide (3) et disposé dans l'appareil (1), qui est conçu pour détecter au moins un composant de l'air ambiant,
un actionneur de micro-fluide (5) connecté en aval du capteur (4) et disposé dans l'appareil (1), qui est conçu pour aspirer, dans la course d'aspiration, du fluide à travers le canal à fluide (3) et à le transporter en direction du capteur (4) et pour transporter, dans la course de refoulement, le fluide aspiré à travers ce même canal à fluide (3) à nouveau en direction de l'ouverture du boîtier (2),
dans lequel le capteur (4) est disposé distant de l'ouverture du boîtier (2), et le volume du canal à fluide (3) entre le capteur (4) et l'ouverture du boîtier (2) est aussi grand ou plus petit que le volume de déplacement de piston que l'actionneur de micro-fluide (5) peut transporter en une seule course d'aspiration,
**caractérisé par le fait que**
le volume de déplacement de piston de l'actionneur de micro-fluide (5) est au moins 2,5 fois supérieur au volume d'un segment de canal à fluide (3a) entre le capteur (4) et l'ouverture du boîtier (2), et
**par le fait que** l'actionneur de micro-fluide (5) est un actionneur à membrane avec un support (8) et une membrane pouvant être déviée (6) y disposée, et que la membrane (6) est précontrainte mécaniquement, de sorte que la membrane (6) soit, dans une position de repos non actionnée, distante du support (8) et se déplace vers le support (8) lorsqu'elle est actionnée.

2. Appareil (1) selon la revendication 1, dans lequel le canal à fluide (3) s'étend entre l'ouverture de boîtier (2) et l'actionneur de micro-fluide (5), et le canal à fluide (3) constitue, ensemble avec le capteur (4) couplé au canal à fluide (3) et l'actionneur de micro-fluide (5), un aménagement étanche aux fluides qui est étanche par rapport à l'intérieur de l'appareil (1).

3. Appareil (1) selon l'une des revendications précédentes, dans lequel le volume de déplacement de piston de l'actionneur de micro-fluide (5) est au moins 10 fois supérieur au volume du segment de canal à fluide (3a) entre le capteur (4) et l'ouverture de boîtier (2).

4. Appareil (1) selon l'une des revendications précédentes, dans lequel le capteur (4) est conçu pour détecter au moins un composant de l'air ambiant du groupe composé de monoxyde de carbone (CO), de dioxyde de carbone (CO₂), d'azote (N₂), de monoxyde de diazote (N₂O), de composés organiques volatiles (VOC), d'humidité et de poussières fines.

5. Appareil (1) selon l'une des revendications précédentes, dans lequel la membrane (6) peut être actionnée de manière piézoélectrique ou électromagnétique ou électrostatique ou au moyen d'actionneurs électro-actifs en polymère ou au moyen d'un élément (7) présentant un alliage à mémoire de forme.

6. Appareil (1) selon l'une des revendications précédentes, dans lequel l'actionneur de micro-fluide (5) est un actionneur à membrane avec une double membrane pouvant être déviée (6).

7. Appareil (1) selon l'une des revendications précédentes, dans lequel la membrane (6), y compris le support (8), présente une hauteur de 0,50 mm ou moins.

8. Appareil (1) selon l'une des revendications précédentes, dans lequel l'appareil (1) présente deux ou plusieurs capteurs (4a, 4b, 4c, 4d) qui sont disposés en série l'un après l'autre dans la direction de circulation, ou dans lequel l'appareil (1) présente deux ou plusieurs capteurs (4a, 4b, 4c, 4d) qui sont disposés parallèlement l'un à l'autre dans la direction de circulation.

9. Appareil (1) selon la revendication 8, dans lequel l'appareil (1) présente deux ou plusieurs actionneurs de micro-fluide (5a, 5b, 5c, 5d), dans lequel un actionneur de micro-fluide (5a, 5b, 5c, 5d) est chaque fois en communication de fluide avec un capteur (4a, 4b, 4c, 4d).

10. Appareil (1) selon l'une des revendications précédentes, dans lequel est disposé, entre l'ouverture du boîtier (2) et le capteur (4) et/ou entre le capteur (4) et l'actionneur de micro-fluide (5), un élément de filtre perméable à l'air qui est conçu pour attraper le liquide condensé dans le fluide aspiré.

11. Appareil (1) selon l'une des revendications précédentes, dans lequel l'appareil est un appareil mobile.

12. Appareil (1) selon la revendication 11, dans lequel l'appareil mobile (1) est un téléphone portable et l'ouverture de boîtier (2) prévue dans le téléphone portable est une ouverture de microphone.

13. Appareil (1) selon la revendication 11 ou 12, dans lequel l'appareil mobile (1) est un téléphone portable avec un moteur d'alarme vibratoire et l'actionneur de micro-fluide (5) est un actionneur à membrane avec une membrane pouvant être déviée (6), dans lequel la membrane (6) peut être actionnée au moyen du moteur d'alarme vibratoire.

14. Appareil (1) selon la revendication 13, dans lequel l'appareil mobile (1) est un téléphone portable ou un téléphone intelligent ou un ordinateur portable ou mobile.

15. Appareil selon l'une des revendications 1 à 10, dans lequel l'appareil est un appareil stationnaire.
